(19) Europäisches Patentamt
European Patent Office
Office européen des brevets

(11) Veröffentlichungsnummer: **0 096 320**
**B1**

(12) **EUROPÄISCHE PATENTSCHRIFT**

(45) Veröffentlichungstag der Patentschrift: **30.04.86**

(51) Int. Cl.⁴: **A 61 K 31/55**

(21) Anmeldenummer: **83105297.2**

(22) Anmeldetag: **28.05.83**

(54) **Verwendung von 5-Phenyl-1,4-benzodoazepinen zur Herstellung von analgetisch wirksamen pharmazeutischen Zubereitungen.**

(30) Priorität: **05.06.82 DE 3221402**

(43) Veröffentlichungstag der Anmeldung: **21.12.83 Patentblatt 83/51**

(45) Bekanntmachung des Hinweises auf die Patenterteilung: **30.04.86 Patentblatt 86/18**

(84) Benannte Vertragsstaaten: **AT CH DE FR GB LI NL SE**

(56) Entgegenhaltungen:
**EP - A - 0 075 855**
**CA - A - 826 159**
**DE - A - 2 221 558**

**Beilsteins Handbuch der organ. Chemie, Julius-Springer-Verlag 1920, Bd. 2, Seite 3**
**Nomenclature of Organic Chemistry, 1979, Pergamon Press, Rule C 403, S. 185**

(73) Patentinhaber: **Kali-Chemie Pharma GmbH, Hans-Böckler-Allee 20 Postfach 220, D-3000 Hannover 1 (DE)**

(72) Erfinder: **Römer, Dietmar, Dr.rer.nat., Lettenweg 111, CH-4123 Allschwil (CH)**
Erfinder: **Ruhland, Michael, Dipl.-Biol., Dr.rer.nat., Bachstelzenweg 14, D-3000 Hannover 61 (DE)**
Erfinder: **Zeugner, Horst, Dipl.-Chem., Dr.rer.nat., Havelweg 10, D-3000 Hannover 73 (DE)**
Erfinder: **Milkowski, Wolfgang, Dipl.-Chem., Dr.rer.nat., Fasanenweg 13, D-3167 Burgdorf (DE)**
Erfinder: **Liepmann, Hans, Dipl.-Chem., Dr.rer.nat., Auf dem Emmerberge 17, D-3000 Hannover (DE)**

(74) Vertreter: **Lauer, Dieter, Dr., c/o Kali-Chemie AG Postfach 220 Hans-Böckler-Allee 20, D-3000 Hannover 1 (DE)**

**EP 0 096 320 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft die Verwendung von 5-Phenyl-1H-2,3-dihydro-1,4-benzodiazepin-Derivaten der allgemeinen Formel I

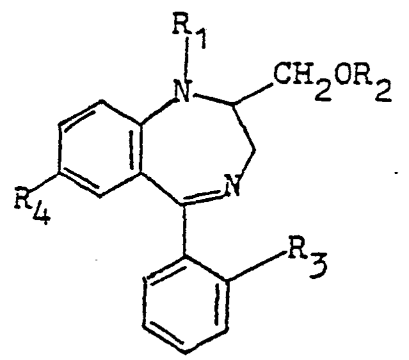

I

worin
$R_1$ Wasserstoff oder Alkyl mit 1-4 Kohlenstoffatomen bedeutet,
$R_2$ Wasserstoff, Alkyl mit 1-4 Kohlenstoffatomen oder Alkanoyl mit 1-4 Kohlenstoffatomen bedeutet,
$R_3$ Wasserstoff oder Halogen bedeutet, und
$R_4$ Halogen bedeutet, oder deren physiologisch verträglichen Säureadditionssalzen als analgetische Wirkstoffe zur Herstellung von analgetisch wirksamen pharmazeutischen Zubereitungen.

In den Verbindungen der Formel I können die niederen Alkyl- oder Alkanoylgruppen 1 bis 4 Kohlenstoffatome enthalten. $R_1$ stellt vorzugsweise Methyl, Äthyl oder Wasserstoff dar. $R_2$ stellt bevorzugt niederes Alkyl, insbesondere Alkyl mit 1 bis 3 Kohlenstoffatomen, bevorzugt Methyl, oder Wasserstoff dar. $R_3$ steht vorzugsweise für Halogen, insbesondere Fluor, Chlor oder Brom, bevorzugt Chlor. Der Halogensubstituent $R_4$ stellt insbesondere Chlor oder Brom, bevorzugt Brom dar. Insbesondere eignen sich das 1-Methyl-7-brom-2-methoxymethyl-5-(2-chlorphenyl)-1H-2,3-dihydro-1,4-benzodiazepin und seine physiologisch verträglichen Säureadditionssalze.

Die als analgetische Wirkstoffe in den erfindungs-gemäßen pharmazeutischen Zubereitungen verwendeten Benzodiazepin-Derivate der Formel I fallen unter in 2-Stel-lung einen substituierten Methylrest tragende 5-Phenyl-1H-2,3-dihydro-1,4-benzodiazepin-Verbindungen welche aus den deutschen Offenlegungsschriften 22 21 558, 23 55 160, 25 20 937 und 31 38 769 sowie dem belgischen Patent 799 001 und der europäischen Patentanmeldung 75 885 bekannt sind und welche das Zentralnervensystem beeinflußende, insbesondere anxiolytische und tranguilisierende Eigenschaften besitzen.

Der Erfindung liegt die Aufgabe zugrunde neue analgetisch wirksame pharmazeutische Zubereitungen mit verbessertem Wirkungsprofil zu entwickeln.

Es wurde nun überraschend gefunden, daß die Verbindungen der Formel I sich durch ein neuartiges Wirkungsprofil auszeichnen und neben den vorgenannten ZNS-Wirkungen auch eine starke analgetische Wirkungskomponente besitzen. Die analgetische Wirkung der Substanzen der Formel I entfaltet sich überraschenderweise bereits in einem Dosisbereich, in welchem sedierende und muskelrelaxierende Effekte noch nicht beobachtet werden können.

Aufgrund dieses günstigen Wirkungsprofils und ihrer guten Verträglichkeit erweisen sich die Verbindungen der Formel I besonders geeignet als wertvolle Wirkstoffe zur Herstellung von schmerzstillenden pharmazeutischen Zubereitungen.

Die analgetische Wirkung und die gute Verträglichkeit der Verbindungen der Formel I, insbesondere das günstige Verhältnis von analgetisch wirksamen Dosen zu Dosen, in welchen unerwünschte sedierende und muskelrelaxierende Eigenschaften beobachtet werden können, lassen sich in pharmakologischen Standardtests an Tieren nachweisen, z.B. durch Vergleich der schmerzhemmenden Wirkung im Arthritisschmerztest und der die allgemeine Aktivität beeinträchtigenden sedativen und muskelrelaxierenden Wirkungen an Ratten.

**Beschreibung der pharmakologischen Versuchsmethoden**

1. Arthritisschmerztest an der Ratte.

Männlichen Ratten vom Stamm OFA mit einem Gewicht von 160 - 180 g werden durch Gaben von 20 mg/kg i.p. Pentobarbitalnatrium anästhesiert. 0,1 ml einer Suspension von Mycobakterium Smegmae (SI043) in Paraffinöl (0,6 mg Mycobakt./0,1 ml Öl) werden intracutan in die linke Hinterpfote injiziert. 14 Tage später, wenn sich besonders in der rechten Hinterpfote eine ausgeprägte sekundäre Arthritis entwickelt hat, werden die Wirkungen der Testsubstanzen untersucht. 30 Minuten vor Verabreichung der Testsubstanzen wird eine Kontrollmessung vorgenommen, indem das Fußgelenk der rechten Hinterpfote dreimal gebeugt wird und die Anzahl der Lautgebungen gezählt wird. Ratten, welche nicht reagieren, werden ausgesondert. 3 Stunden nach

oraler Gabe der Testsubstanzen wird die Beugeprozedur wiederholt. Tiere, welche entweder nur einmal oder überhaupt nicht Laut geben, werden als geschützt gegen Schmerzen angesehen. Als $ED_{50}$ wird die Dosis bezeichnet, welche einen Schutz in 50 % der behandelten Tiere erzeugt.

2. Bestimmung der Hemmung der lokomotorischen Explorationsaktivität nach der Methode von Janssen (Psychopharmakol. 1 (1961), 141 - 146).

Männlichen Ratten vom Typ Spraque-Dawley wird die Testsubstanz in Form einer Suspension in 2 %iger Tyloselösung 1 Stunde vor Versuchsbeginn oral appliziert. Es werden 6 Rattenpaare pro Dosis eingesetzt. Die Ratten werden paarweise in eine für sie unbekannte Umgebung gegeben und ihre lokomotorische Aktivität in der Erkundungs- und Neugierphase während der ersten 30 Sek. wird von 2 Beobachtern registriert. Die Aktivität wird nach einem Punktsystem bewertet, wobei die höchstmögliche Punktzahl für 6 Paare 18 Punkte beträgt. Als $ED_{50}$ wird diejenige Dosis bezeichnet, welche 9 Punkte für die 6 Paare ergibt.

In der folgenden Tabelle werden die nach den vorstehend beschriebenen Testmethoden für das 1-Methyl-7-brom-2-methoxymethyl-5-(2-chlorphenyl)-1H-2,3-dihydro-1,4-benzodiazepin (Verbindung Nr. 1, als Hydrochlorid) und für das 1-Methyl-7-chlor-2-methoxymethyl-5-(2-chlor-phenyl)-1H-2,3-dihydro-1,4-benzodiazepin (Verbindung Nr. 2, als Hydrochlorid) erhaltenen Werte mit den für die Vergleichssubstanz Diazepam (7-Chlor-1-methyl-5-phenyl-1,3-dihydro-2H-1,4-benzodiazepin-2-on) erhaltenen Werten verglichen.

| Test-substanz | Hemmung des Arthritis-schmerzes an der Ratte $ED_{50}$ mg/kg p.o. | Hemmung der lolomotor. Explorations-aktivität an der Ratte $ED_{50}$ mg/kg p.o. | akute Toxizität an der Maus $LD_{50}$ mg/kg p.o. |
|---|---|---|---|
| Verbindung | | | |
| 1 | 4,8 | 56,2 | 2390 |
| 2 | 10,0 | 55,5 | 1470 |
| Diazepam | 6,2 | 11,5 | 825 |

Aus den Testergebnissen geht klar hervor, daß die Verbindungen der Formel I eine gute analgetische Wirkung besitzen, im Gegensatz zu Diazepam eine negative Beeinflussung des normalen lokomotorischen Neugierverhaltens jedoch erst bei wesentlich höheren Dosierungen bewirken.

Als Wirkstoffe zur Herstellung von analgetisch wirksamen pharmazeutischen Zubereitungen können die Verbindungen der Formel I in Form der freien Basen oder ihrer physiologisch verträglichen Säureadditionssalze eingesetzt werden. Als physiologisch verträgliche Säureadditonssalze der Verbindungen der Formel I eignen sich beispielsweise deren Salze mit anorganischen Säuren wie Chlorwasserstoffsäure, Bromwasserstoffsäure, Schwefelsäure oder Phosphorsäure, oder Salze mit organischen Säuren wie beispielsweise Maleinsäure, Fumarsäure, Essigsäure, Benzoesäure, Methansulfonsäure, Cyclohexylaminosulfonsäure, Milchsäure, Weinsäure oder Phenylessigsäure.

Die zu verwendenden Dosen können individuell verschieden sein und variieren naturgemäß je nach Art des zu behandelnden Zustandes, der verwendeten Substanz und der Applikationsform. Z.B. werden parenterale Formulierungen im allgemeinen weniger Wirkstoff enthalten als orale Präparate. Im allgemeinen eignen sich zur Applikation an Menschen und größeren Säugetieren jedoch Arzneiformen mit einem Wirkstoffgehalt von 0,25 bis 25, insbesondere 5 bis 15 mg Wirkstoff pro Einzeldosis.

Die Verbindungen der Formel I können erfindungs-gemäß zusammen mit üblichen pharmazeutischen Hilfsund/oder Trägerstoffen in festen oder flüssigen pharmazeutischen Zubereitungen enthalten sein. Als Beispiele fester Präparate seien oral applizierbare Präparate wie Tabletten, Kapseln, Pulver, Granulate oder Dragees genannt oder auch Suppositorien. Feste Präparate können pharmazeutisch übliche anorganische und/oder organische Trägerstoffe wie z.B. Talkum, Milchzucker oder Stärke neben pharmazeutisch üblichen Hilfsmitteln, beispielsweise Gleitmitteln wie Magnesiumstearat oder Tablettensprengmitteln, enthalten. Flüssige Präparate wie Lösungen, Suspensionen oder Emulsionen können die üblichen Verdünnungsmittel wie Wasser, Öle oder Vaseline ® und/oder Suspensionsmittel wie Polyoxyäthylenglykol und dergl. enthalten. Es können zusätzlich weitere Hilfsstoffe zugegeben werden, wie z.B. Konservierungsmittel, Stabilisierungsmittel, Geschmackskorrigenzien und dergl.

Gewünschtenfalls können feste orale Arzneiformen auch die Freisetzung des Wirkstoffes verzögernde Stoffe wie z.B. Polyvinylacetate, Acrylat- oder Methacrylatcopolymere, höhere Fettalkohole und andere wachsartige

Substanzen enthalten.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die Verbindungen der Formel I sind aus den DOS 22 21 558, 23 55 160, 25 20 957 und 31 38 769 sowie dem belgischen Patent 799 001 und der europäischen Patentanmeldung 75 885 bekannt oder können nach in diesen Patenten beschriebenen Verfahren hergestellt werden.

Insbesondere können durch Acylierung entsprechender 2-Hydroxy-1,4-diaminopropane hergestellte Acyldiaminoverbindungen der Formel II

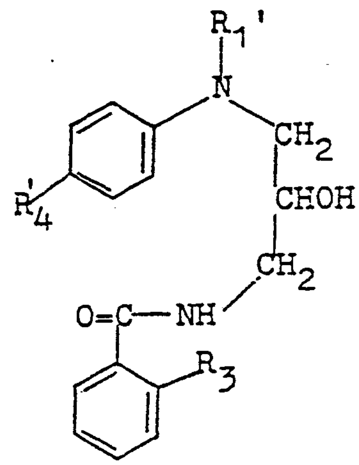

II

worin $R_3$ obige Bedeutung besitzt, $R_4'$ Halogen oder Wasserstoff bedeutet und $R_1'$ niederes Alkyl bedeutet, in an sich bekannter Weise mit Phosphoroxidchlorid cyclisiert werden zu einem Gemisch der isomeren Verbindungen der Formel IIIa und IIIb

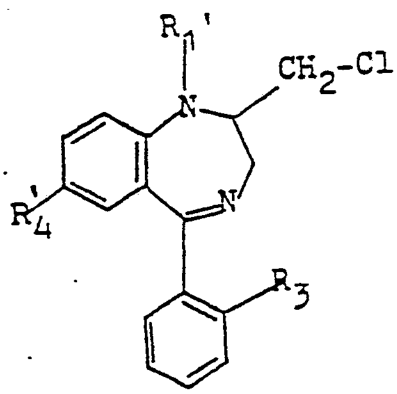

IIIa

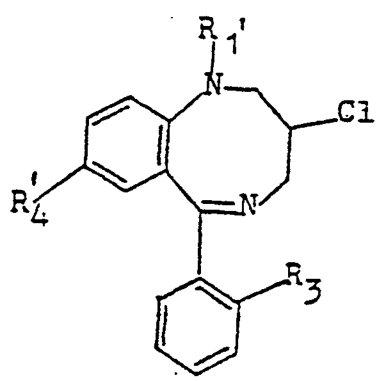

IIIb

worin $R_1'$, $R_3$ und $R_4'$ obige Bedentung besitzen, und dieses Gemisch anschließend in an sich bekannter Weise mit einem nukleophilen Reagenz, z.B. einem Alkalimetallhydroxid oder -alkoholat zu Alkoholen oder Äthern der Formel I ($R_2$ = H oder niederes Alkyl) umgesetzt werden und erhaltene Alkohole anschließend gewünschtenfalls in Ester ($R_2$ = niederes Alkanoyl) überführt werden.

Sofern $R_4'$ für Wasserstoff steht, kann dieses auf an sich bekannte Weise in den Verbindungen der Formel IIIa und IIIb, oder gewünschtenfalls auch nachträglich in den Endprodukten der Formel I, in Chlor oder Brom überführt werden, z.B. durch Behandeln mit Chlor- oder Bromsuccinimid.

Zur Herstellung von Verbindungen der Formel I worin $R_1$ Wasserstoff bedeutet, werden zweckmäßigerweise Verbindungen der Formel IIIa, worin $R_1'$ Methyl und $R_4$ Halogen bedeuten, durch Behandlung mit Jodwasserstoff demethyliert und die demethylierten Produkte durch Behandeln mit einer starken Base, beispielsweise einen Alkalimetallalkoholat oder -hydrid, in einem inerten Lösungsmittel bei erhöhten Temperaturen cyclisiert zu Verbindungen der Formel IV

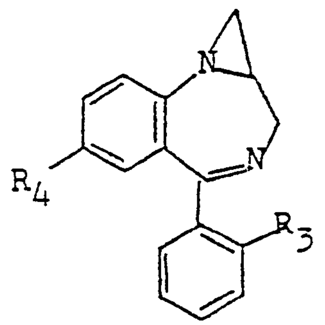

IV

worin $R_3$ und $R_4$ obige Bedeutung besitzen, und diese in Gegenwart einer Lewissäure mit einem niederen Alkohol oder einer niederen aliphatischen Carbonsäure umgesetzt zu Verbindungen der Formel Ia

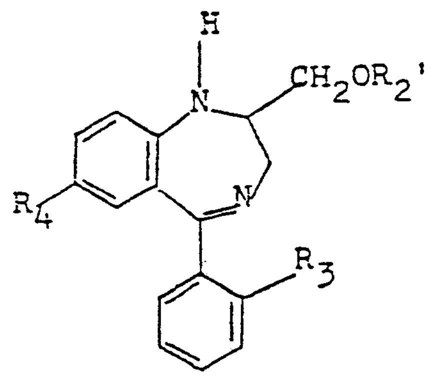

Ia

worin $R_3$ und $R_4$ obige Bedeutung besitzen und $R_2'$ niederes Alkyl oder niederes Alkanoyl bedeutet, und gewünschtenfalls Ester ($R_2$ = niederes Alkanoyl) zu Alkoholen ($R_2$ = H) der Formel I hydrolisiert.

Die nachfolgenden Beispiele erläutern die Herstellung der erfindungsgemäßen pharmazeutischen Zubereitungen. Sie sollen jedoch den Umfang der Erfindung in keiner Weise beschränken.

**Beispiel 1: Tabletten**

Man stellt Tabletten in folgender Zusammensetzung pro Tablette her:

| | |
|---|---|
| 1-Methyl-7-brom-2-methoxymethyl-5-(2-chlorphenyl)—1H-2,3-dihydro-1,4-benzodiazepinhydrochlorid | 10 mg |
| Maisstärke | 60 mg |
| Milchzucker | 145 mg |
| Gelatine (10 %ige Lösung) | 6 mg |

Der Wirkstoff, die Maisstärke und der Milchzuckerwerden mit der 10 %igen Gelatinelösung eingedickt. Die Paste wird zerkleinert und das entstandene Granulat wird auf ein geeignetes Blech gebracht und bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 g verpreßt.

**Beispiel 2 Dragees**

Die nach Beispiel 1 hergestellten Tablettenkerne werden in an sich bekannter Weise dragiert und die erhaltenen Dragees mit Hilfe von Bienenwachs poliert.

**Beispiel 3: Tabletten**

Man stellt Tabletten in folgender Zusammensetzung pro Tablette her:

| | |
|---|---|
| 7-Brom-2-hydroxymethyl-5-(2-chlorphenyl)-1H 2,3-dihydro-1,4-benzodiazepin-hydrochlorid | 5 mg |
| Maisstärke | 60 mg |
| Milchzucker | 150 mg |
| Gelatine (10 %ige Lösung) | 6 mg |
| Magnesiumstearat | 5 mg |
| Maisstärke | 9 mg |

Die Bestandteile werden wie in Beispiel 1 beschrieben zu Tabletten von 240 mg Gewicht verarbeitet.

**Beispiel 4: Kapseln**

Man stellt Kapseln mit folgender Zusammensetzung pro Kapsel her:

| | |
|---|---|
| 1-Methyl-7-brom-2-methoxymethyl-5-(2-chlor phenyl)-1H-2,3-dihydro-1,4-benzodiazepin | 10 mg |
| Mannit | 175 mg |
| Talkum | 12 mg |
| kolloidale Kieselsäure | 1 mg |
| Magnesiumstearat | 2 mg |
| | 200 mg |

Die gesiebten Substanzen werden gemischt und daserhaltene Pulver in Portionen von 200 mg in Kapseln abgefüllt.

**Beispiel 5: Suppositorien**

Man stellt Suppositorien in folgender Zusammensetzung pro Suppositorium her:

| | |
|---|---|
| 1-Methyl-7-chlor-2-methoxymethyl-5-(2-chlorphenyl)-1H-2,3-dihydro-1,4-benzodiazepinhydrochlorid | 25 mg |
| Kakaobutter | 1975 mg |

Der Wirkstoff und die fein geriebene Suppositoriengrundmasse werden gründlich gemischt und dann geschmolzen. Aus der durch Rühren homogen gehaltenen Schmelze werden Suppositorien von 2 g gegossen.

**Beispiel 6: Injektionslösung**

Man stellt eine Injektionslösung in folgender Zusammensetzung pro ml her:

| | |
|---|---|
| 1-Methyl-7-brom-2-methoxymethyl-5-(2-chlor phenyl)-1H-2,3-dihydro-1,4-benzodiazepin | 5 mg |
| Dimethylacetamid | 10 mg |
| Propylenglykol | 50 mg |
| Benzylalkohol | 1,4 mg |
| Äthanol | 10 mg |
| Wasser für Injektionszwecke ad | 1 ml |

Der Wirkstoff wird in Dimethylacetamid gelöst undmit Benzylalkohol, Propylenglykol, Äthanol und Wasser versetzt. Man filtriert durch einen Kerzenfilter, füllt in geeignete Ampullen, verschließt und sterilisiert.

**Beispiel 7: Tropfen**

Man stellt eine Tropflösung mit folgender Zusammensetzung pro ml her:

| | |
|---|---|
| 1-Methyl-7-brom-2-methoxymethyl-5-(2-chlorphenyl)-1H-2,3-dihydro-1,4-benzodiazepinhydrochlorid | 10 mg |
| Äthanol | 300 mg |
| Propandiol-(1,2) | 300 mg |
| Sorbitol | 350 mg |
| 1 n NaOH zur Einstellung des pH auf 6,5 | q.s. |
| Geschmackskorrigenz (Orangengeschmack) | q.s. |
| Demineralisiertes Wasser | ad 1 ml |

Der Wirkstoff wird in den flüssigen Bestandteilengelöst und die Lösung auf Tropffläschchen abgefüllt.

## Patentansprüche

1. Verwendung von 5-Phenyl-1H-2,3-dihydro-1,4-benzodiazepin-Derivaten der allgemeinen Formel I

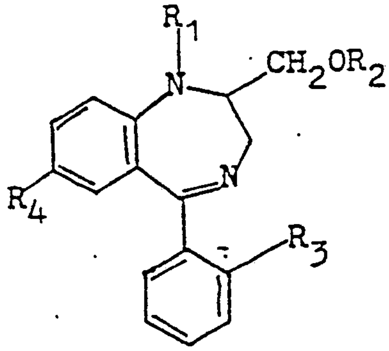

I

worin
$R_1$ Wasserstoff oder Alkyl mit 1-4 Kohlenstoffatomen bedeutet,
$R_2$ Wasserstoff, Alkyl mit 1-4 Kohlenstoffatomen oder Alkanoyl mit 1-4 Kohlenstoffatomen bedeutet,
$R_3$ Wasserstoff oder Halogen bedeutet, und
$R_4$ Halogen bedeutet, oder deren verträglichen Säureadditionssalzen zur Herstellung von analgetisch wirksamen pharmazeutischen Zubereitungen.

2. Verwendung gemäß Anspruch 1, dadurch gekennzeichnet, daß Verbindungen der Formel I verwendet werden, worin $R_1$ Alkyl mit 1-4 Kohlenstoffatomen bedeutet und $R_2$, $R_3$ und $R_4$ obige Bedeutung besitzen.

3. Verwendung gemäß Anspruch 1, dadurch gekennzeichnet, daß Verbindungen der Formel I verwendet werden, worin $R_4$ Chlor oder Brom bedeutet und $R_1$, $R_2$ und $R_3$ obige Bedeutung besitzen.

4. Verwendung gemäß Anspruch 1 dadurch gekennzeichnet, daß Verbindungen der Formel I verwendet werden, worin $R_2$ Alkyl mit 1-4 Kohlenstoffatomen bedeutet und $R_1$, $R_3$ und $R_4$ obige Bedeutung besitzen.

5. Verwendung gemäß Anspruch 1, dadurch gekennzeichnet, daß Verbindungen der Formel I verwendet werden, worin $R_1$ Wasserstoff oder Methyl bedeutet, $R_2$ Wasserstoff oder Alkyl mit 1 bis 3 Kohlenstoffatomen bedeutet, $R_3$ Halogen bedeutet und $R_4$ Brom oder Chlor bedeutet.

6. Verwendung gemäß Anspruch 5, dadurch gekennzeichnet, daß eine Verbindung der Formel I verwendet wird, worin $R_1$ und $R_2$ je Methyl, $R_3$ Chlor und $R_4$ Brom bedeuten.

7. Verwendung gemäß Anspruch 5, dadurch gekennzeichnet, daß eine Verbindung der Formel I verwendet wird, worin $R_1$ und $R_2$ je Methyl und $R_3$ und $R_4$ je Chlor bedeuten.

8. Verfahren zur Herstellung von analgetisch wirksamen pharmazeutischen Zubereitungen gemäß Anspruch 1, dadurch gekennzeichnet, daß man 5-Phenyl-1H-2,3-dihydro-1,4-benzodiazepin-Derivate der allgemeinen Formel I worin $R_1$, $R_2$, $R_3$ und $R_4$ die in Anspruch 1 genannte Bedeutung besitzen, oder deren physiologisch verträgliche Säureadditionssalze zusammen mit üblichen pharmazeutischen Hilfs- und/oder Trägerstoffen in eine geeignete Arzneiform überführt.

**Revendications**

1. Utilisation de dérivés de 5-phenyl-1H-2,3-dihydro-1,4-benzodiazépines de formule générale I

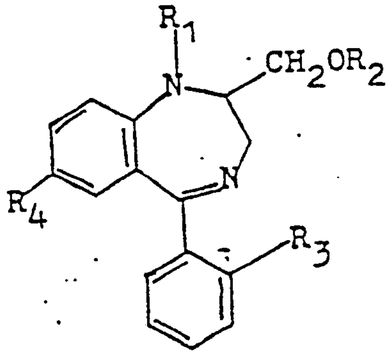

I

dans laquelle
$R_1$ est l'hydrogène ou un radical alkyle ayant 1-4 atomes de carbone,
$R_2$ est l'hydrogène, un radical alkyle ayant 1-4 atomes de carbone ou alcanoyle ayant 1-4 atomes de carbone,

$R_3$ est l'hydrogène ou un halogène, et
$R_4$ est un halogène,
ou de leurs sels d'addition avec un acide, acceptables, pour préparer des compositions pharmaceutiques à effet analgésique.

2. Utilisation selon la revendication 1, caractérisée en ce qu'on utilise des composés de formule I dans laquelle $R_1$ est un radical alkyle ayant 1-4 atomes de carbone et $R_2$, $R_3$ et R ont la signification ci-dessus.

3. Utilisation selon la revendication 1, caractérisée en ce que l'on utilise des composés de formule I dans laquelle $R_4$ est le chlore ou le brome, et $R_1$, $R_2$ et $R_3$ ont la signification ci-dessus.

4. Utilisation selon la revendication 1, caractérisée en ce que l'on utilise des composés de formule I dans laquelle $R_2$ est un radical alkyle ayant 1-4 atomes de carbone et $R_1$, $R_3$ et $R_4$ ont la signification ci-dessus.

5. Utilisation selon la revendication 1, caractérisée en ce que l'on utilise des composés de formule I dans laquelle $R_1$ est l'hydrogène ou le radical méthyle, $R_2$ est l'hydrogène ou un radical alkyle ayant 1 à 3 atomes de carbone, $R_3$ est un halogène et $R_4$ est le brome ou le chlore.

6. Utilisation selon la revendication 5, caractérisée en ce qu'on utilise un composé de formule I dans laquelle $R_1$ et $R_2$ sont chacun le radical méthyle, $R_3$ est le chlore et $R_4$ est le brome.

7. Utilisation selon la revendication 5, caractérisée en ce que l'on utilise un composé de formule I dans laquelle $R_1$ et $R_2$ sont chacun le radical méthyle, et $R_3$ et $R_4$ sont chacun le chlore.

8. Procédé pour la préparation de compositions pharmaceutiques à effet analgésique selon la revendication 1, caractérisé en ce qu'on convertit en une forme médicamenteuse appropriée des dérivés de 5-phényl-1H-2,3-dihydro-1,4-benzodiazépine de formule générale I, dans laquelle $R_1$, $R_2$, $R_3$ et $R_4$ ont la signification donnée dans la revendication 1, ou leurs sels d'addition avec un acide physiologiquement acceptables, avec des matières auxiliaires et/ou excipients pharmaceutiques habituels.

**Claims**

Use of 5-phenyl-1H-2,5-dihydro-1,4-benzodiazepine derivatives of the general Formula I

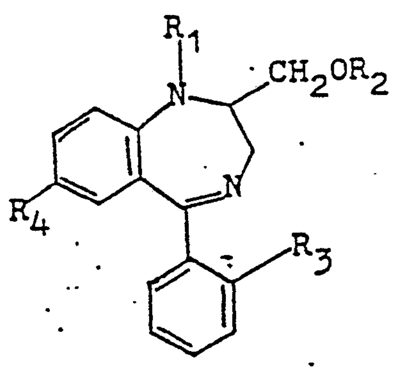

I

in which
$R_1$ denotes hydrogen or alkyl with 1-4 carbon atoms,
$R_2$ denotes hydrogen, alkyl with 1-4 carbon atoms or alkanoyl with 1-4 carbon atoms,
$R_3$ denotes hydrogen or halogen, and
$R_4$ denotes halogen, or their compatible acid addition salts for the manufacture of analgesically effective pharmaceutical preparations.

2. Use according to Claim 1, characterized in that compounds of Formula I are used, in which $R_1$ denotes alkyl with 1-4 carbon atoms and $R_2$, $R_3$ and $R_4$ have the above meaning.

3. Use according to Claim 1, characterised in that compounds of Formula I are used, in which $R_4$ denotes chlorine or bromine and $R_1$, $R_2$ and $R_3$ have the above meaning.

4. Use according to Claim 1 characterized in that compounds of Formula I are used, in which $R_2$ denotes alkyl with 1-4 carbon atoms and $R_1$, $R_3$ and $R_4$ have the above meaning.

5. Use according to Claim 1, characterized in that compounds of Formula I are used, in which $R_1$ denotes hydrogen or methyl, $R_2$ denotes hydrogen or alkyl with 1 to 3 carbon atoms, $R_3$ denotes halogen and $R_4$ denotes bromine or chlorine.

6. Use according to Claim 5 characterized in that a compound of Formula I is used, in which $R_1$ and $R_2$ each denote methyl, $R_3$ denotes chlorine and $R_4$ denotes bromine.

7. Use according to Claim 5, characterized in that a compound of Formula I is used, in which $R_1$ and $H_2$ each denote methyl and $R_3$ and $R_4$ each denote chlorine.

8. Method for the manufacture of analgesically effective pharmaceutical preparations according to Claim 1, characterized in that 5-phenyl-1H-2,3-dihydro-1,4-benzodiazepine derivatives of the general Formula I, in which

$R_1$, $R_2$, $R_3$ and $R_4$ have the meaning indicated in Claim 1, or their physiologically compatible acid addition salts together with conventional pharmaceutical adjuvant- and/or carrier substances are converted into a suitable form of medicament.